# EUROPEAN PATENT APPLICATION

(11) **EP 4 227 404 A1**
(43) Date of publication of application: **16.08.2023**
(21) Application number: 21878059.1
(22) Date of filing: 08.10.2021
(51) Int. Cl.: C12N 5/077, C12N 5/00, C12N 11/08, C12M 1/00, C12M 1/12, A61K 35/32, A61P 19/00

(54) **OSTEOBLASTS DIFFERENTIATED FROM MESENCHYMAL STEM CELLS AND COMPOSITION FOR TREATING BONE DISEASE COMPRISING SAME**

(30) Priority: 08.10.2020 KR 20200130138
(71) Applicant: Cefo Co., Ltd., Seoul 03150 (KR)
(72) Inventor: PARK, Hyun Sook, Seoul 07504 (KR); LEE, Yeon Kyung, Yongin-si, Gyeonggi-do 17095 (KR); GO, Eun Jin, Anyang-si, Gyeonggi-do 13979 (KR); KIM, Ha Jin, Seoul 08296 (KR); LEE, Sun Ray, Seoul 06723 (KR); KWON, Ah Reum, Seoul 05021 (KR)
(74) Representative: Dehns
(86) International application number: PCT/KR2021/013900
(87) International publication number: WO 2022/075809

(57) **Abstract**

The present invention relates to a method for differentiating mesenchymal stem cells into osteoblasts, a cell therapeutic agent for treating bone disease including osteoblasts differentiated by the method and a method for producing the same. In addition, the present invention relates to a method for treating bone disease, including the step of administering the osteoblasts obtained by the method to a patient with bone disease.

The differentiation method according to the present invention can stably and rapidly differentiate mesenchymal stem cells into osteoblasts. The differentiated osteoblasts have excellent blood vessel-forming ability and excellent bone-forming ability. Accordingly, the method for differentiating stem cells into osteoblasts and the osteoblasts obtained by the method, according to the present invention, can be effectively used as a cell therapeutic agent or treatment method related to bone disease.

## Description

### CROSS-REFERENCE TO RELATED APPLICATION

This application claims priority to and the benefit of Korean Patent Application No. 10-2020-0130138, filed on October 8, 2020, the disclosure of which is incorporated herein by reference in its entirety.

### [Technical Field]

The present invention relates to a method for differentiating mesenchymal stem cells into osteoblasts, a cell therapeutic agent for treating bone disease including osteoblasts differentiated by the method and a method for producing the same.

In addition, the present invention relates to a method for treating bone disease, including the step of administering the osteoblasts obtained by the method to a patient with bone disease.

### [Background Art]

Stem cells collectively refer to undifferentiated cells having pluripotency that can be differentiated into various cells, and the stem cells can be differentiated into specific cells by a specific differentiation factor and/or environment. The types of stem cells include embryonic stem cells, embryonic germ cells, adult stem cells, cancer stem cells and the like. Recently, studies have been actively conducted to treat various diseases including regeneration of damaged tissues, cartilage damage diseases, diabetes, leukemia, neurological diseases, heart disease, extra-spinal cord injury, fibrotic disorders or the like, by using stem cells capable of differentiating into various cells, and accordingly, studies have been attempted to differentiate stem cells into specific cells. In addition, induced pluripotent stem cells (iPS), which are made of stem cells through dedifferentiation of cells that have been differentiated, have also been used for cell differentiation. In particular, mesenchymal stem cells (MSCs) are multipotent stem cells having the ability to differentiate into various mesodermal cells including bone, cartilage, fat and muscle cells. Due to this ability, mesenchymal stem cells are valuably considered as therapeutic agents in the field of bone disease and tissue damage regenerative medicine. Regenerative medicine is aimed at regenerating a damaged site by activating the unique recovery mechanism of a tissue or organ that has not been recovered or replacing a damaged tissue in the past, and thus includes an attempt to cultivate a tissue or organ that cannot be cured by the human body in a laboratory and safely transplant or inject the tissue or organ into the human body. As a treatment method based on regenerative medicine, stem cell therapeutic agents using the self-renewal ability and differentiation ability of stem cells have attracted attention as next-generation therapeutic agents.

However, due to a risk factor caused by various factors such as the origin of stem cells used, the site of origin, the degree of culture, the degree of differentiation and the like, there is a difficulty in permission due to strict criteria for safety and effectiveness. Further, in order to differentiate and commercially use stem cells having pluripotency that can be differentiated into various cells, mass production is required, but it is difficult to rapidly and safely differentiate stem cells into osteocytes.

For the method of differentiating stem cells into specific cells, Korean Patent No. 10-2016-0034541 discloses a method for differentiating stem cells into osteocytes in a porous membrane coated with a hydrogel by using the sol-gel phase transition, and Korean Patent No. 10-2018-0114307 discloses a method of containing hexanoyl glycol chitosan in a medium to promote the differentiation of mesenchymal stem cells. US Patent No. 8,580,757 B2 discloses a method of using miRNA or siRNA as a method of adjusting the differentiation of mesenchymal stem cells. As described above, various attempts have been made to differentiate mesenchymal stem cells into osteocytes, but the situation is that the method for rapidly and massively differentiating osteocytes in stem cells without adding an expensive composition to a differentiation medium without administering an external substance to the stem cells is still not developed.

However, the situation is that additional studies on the method for stably and rapidly differentiating and culturing osteoblasts from stem cells are still required so as to be utilized as a cell therapeutic agent for bone-related diseases.

### [Disclosure]

### [Technical Problem]

Accordingly, the inventors of the present invention created the present invention by contemplating an optimal method for preparing a therapeutic agent for osteocytes differentiated from mesenchymal stem cells as one of the regenerative medical methods for treating bone disease. The inventors of the present invention intend to provide a method for producing osteoblasts for preparing a cell therapeutic agent for treating bone disease. When the differentiation method of the present invention is used, it can stably and rapidly differentiate into osteoblasts compared to the existing method for differentiating stem cells for cell therapeutic agents, and the present invention was completed by confirming that the osteoblasts have very excellent bone-reconstruction efficacy.

Therefore, it is an object of the present invention to provide a method for differentiating mesenchymal stem cells into osteoblasts, a cell therapeutic agent for treating bone disease including osteoblasts differentiated by the method and a method for producing the same.

### [Technical Solution]

The present invention provides a method for differentiating mesenchymal stem cells into osteoblasts, including the steps of i) coating an air-permeable polymer membrane with surfactant bubbles; ii) inoculating mesenchymal stem cells into the bubbles of step i) at a density of 1 × 10³ to 1 × 10⁵ cells/cm²; iii) differentiating the mesenchymal stem cells of step ii) into osteoblasts in a differentiation medium; and iv) isolating and obtaining osteoblasts differentiated in step iii).

According to a preferred exemplary embodiment of the present invention, the surfactant of step i) may be a poloxamer.

According to a preferred exemplary embodiment of the present invention, the bubbles of step i) may be produced by including the step of generating bubbles by adding a surfactant on the air-permeable polymer membrane and moving.

According to a preferred exemplary embodiment of the present invention, the mesenchymal stem cells of step ii) may be derived from any one or more selected from the group consisting of umbilical cord, umbilical cord blood, placenta, amniotic membrane, bone marrow, fat, hair follicle, tooth, pulp and skin dermis.

In addition, the present invention provides osteoblasts obtained by the method.

According to a preferred exemplary embodiment of the present invention, the osteoblasts may have higher expression levels of connexin 43 (CX43), Runt-related transcription factor 2 (RUNX2) and collagen type 1A1 (COL1A1) than undifferentiated stem cells and mature osteocytes; higher expression levels of angiopoietin 1 (ANGPT1) and alkaline phosphatase (AP) than undifferentiated stem cells; and lower expression levels of osterix (OSX), osteocalcin (OCN) and osteopontin (OPN) than mature osteocytes.

According to a preferred exemplary embodiment of the present invention, the osteoblasts may have a lower expression level of Ki-67 than undifferentiated stem cells.

In addition, the present invention provides a cell therapeutic agent for treating bone disease, including osteoblasts obtained by the method.

According to a preferred exemplary embodiment of the present invention, the osteoblasts may have higher expression levels of connexin 43 (CX43), Runt-related transcription factor 2 (RUNX2) and collagen type 1A1 (COL1A1) than undifferentiated stem cells and mature osteocytes; higher expression levels of angiopoietin 1 (ANGPT1) and alkaline phosphatase (AP) than undifferentiated stem cells; and lower expression levels of osterix (OSX), osteocalcin (OCN) and osteopontin (OPN) than mature osteocytes.

According to a preferred exemplary embodiment of the present invention, the osteoblasts may have a lower expression level of Ki-67 than undifferentiated stem cells.

According to a preferred exemplary embodiment of the present invention, the bone disease may be any one or more selected from the group consisting of fracture, femoral head bone necrosis, spinal union, delayed union or nonunion, osteoporosis, osteonecrosis, pseudoarthrosis, Paget's disease and osteodystrophy.

In addition, the present invention provides a method for treating bone disease, including the step of administering osteoblasts obtained by the method to a patient with bone disease.

As used herein, the term "mesenchymal stem cell" is used synonymously with multipotent undifferentiated cells, and it means various mesenchymal cells including adipocytes, osteoblasts, chondrocytes, cardiac cells or muscle cells, or adult stem cells that have the ability to differentiate into ectodermal cells such as neurons. In addition, it is free from cancer and ethical issues, which are common problems with embryonic stem cells, and may not cause immune rejection after transplantation.

As used herein, the term "osteoblast" is a cell that makes osteocytes of vertebrates and is also called osteoblastocytus. Bone is made by synthesizing and secreting bone matrix, and sometimes it is buried in the bone tissue it has made and becomes normal bone cells by itself. In addition, substances such as Ca and Mg ions necessary for bone can be deposited on the bone to calcify the bone tissue. Depending on the difference and activity of the internal material, the resting phase and the formation phase are divided, and although the division ability is great, the number thereof decreases in older bones.

As used herein, the term "bone disease" refers to a state in which damage occurs to the bone, the composition and density of the bone change, and fractures are easy to occur. Bone is the hardest tissue in the body that maintains the stability of the skeletal system, and it is used as a lever for muscles and serves to protect internal organs as well as to store minerals such as calcium and magnesium. Most of these bone diseases are caused by trauma such as fractures, or metabolic diseases such as avascular necrosis and osteoporosis, and regardless of the cause, there is a problem with the mechanical support of the body, which not only reduces motor performance but also causes persistent pain due to the formation of trachea. In the present specification, bone disease includes osteoporosis, osteonecrosis, pseudoarthrosis, Paget's disease or osteodystrophy.

The term "osteoporosis" refers to a condition in which bone strength is weakened due to a decrease in the amount of bone and a change in the quality of the bone, resulting in a high probability of fracture, and it is mainly caused by heredity, early menopause, excessive diet or steroid drugs.

The term "osteonecrosis" is a disease in which bone tissue dies due to the lack of blood supply to the bones. It can occur anywhere on the body, but most commonly occurs in the upper thigh (thigh bone), upper arm, shoulder, knee, spine or the like.

The term "pseudoarthrosis" is also called a false joint, and after a bone is broken, the site does not stick well (non-union) and moves like a joint. It can be caused by an error in the treatment process after a fracture or a bacterial infection at the fracture site.

The term "Paget's disease" is a localized bone disease in which the skeletal system in various regions is invaded while bone remodeling, which is a process by which new bones are created, grown and resorbed, appears excessively. It usually occurs in the pelvis, thigh or skull.

The term "osteogenesis imperfecta" is a generic term for symptoms in which bones are easily broken without any specific cause due to congenital weakness of the bones, and is also referred to as osteodysplasia.

As used herein, the term "differentiation" refers to a phenomenon in which the structure or function of cells is specialized to each other during division, proliferation and growth, that is, the change in form or function of cells, tissues and the like of living organisms to perform a given task. For example, a state in which there is a qualitative difference between parts of any biological system that was first homologous in the onset of a subject or a state in which the biological system is divided into partial systems that can be qualitatively distinguished as a result thereof is referred to as differentiation.

As used herein, the term "cell therapeutic agent" is a drug used for the purpose of treatment, diagnosis or prevention of cells and tissues prepared by separation, culturing and special manipulation from a subject, and in order to restore the function of a cell or tissue, living autologous, allogeneic or xenogeneic cells may be proliferated *in vitro,* or may be used through a series of actions such as changing the biological characteristics of the cells by another method.

As described above, the conventional cell therapeutic agent using stem cells has been difficult to commercialize publicly due to problems such as high cost, and particularly, there are disadvantages such as time and cost required to differentiate stem cells into osteoblasts.

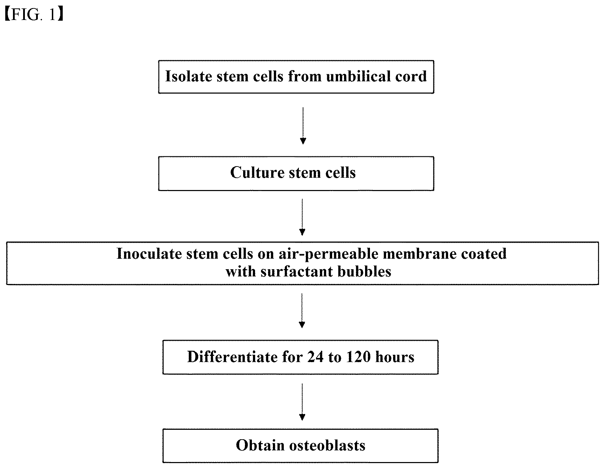

On the other hand, the differentiation method according to the present invention can stably and rapidly differentiate stem cells at the early stage of subculture into osteoblasts in a short period of time. In the conventional stem cell differentiation method, stem cells that are subcultured 8 times or more should be differentiated for about 20 days or more to obtain osteoblasts, whereas in the present invention, when stem cells that are subcultured 5 times are differentiated only for about 3 days, differentiated osteoblasts can be obtained (FIG. 1). In particular, when the differentiation method of the present invention is used, all of the stem cells can be differentiated into osteoblasts without response variation according to the donor of the stem cells, and thus can be used as an allogeneic cell therapeutic agent.

Accordingly, the present invention may provide a method for differentiating mesenchymal stem cells into osteoblasts or osteoblasts differentiated by the method, including the steps of:
i) coating an air-permeable polymer membrane with surfactant bubbles;
ii) inoculating mesenchymal stem cells into the bubbles of step i) at a density of 1 × 10³ to 1 × 10⁵ cells/cm²;
iii) differentiating the mesenchymal stem cells of step ii) into osteoblasts in a differentiation medium; and
iv) isolating and obtaining osteoblasts differentiated in step iii).

The polymer membrane of step i) may be a porous membrane of a hyper flask.

The density of step ii) may be preferably 1 × 10³ to 1 × 10⁵ cells/cm², and more preferably, 1 × 10⁴ cells/cm².

According to a preferred exemplary embodiment of the present invention, the surfactant in step i) may be a poloxamer. Surfactants other than poloxamer are cytotoxic (Example 4) and are not suitable for the present invention. The poloxamer may be any one or more selected from the group consisting of poloxamer 184, poloxamer 185, poloxamer 188, poloxamer 124, poloxamer 237, poloxamer 338 and poloxamer 407.

The surfactant in step i) may be 10% surfactant at most.

The bubbles in step i) may be generated by adding a surfactant on the air-permeable polymer membrane and moving. For example, the bubbles in step i) may be generated by shaking the surfactant on the air-permeable polymer membrane or may be generated through pipetting, but is not limited thereto.

According to a preferred exemplary embodiment of the present invention, the mesenchymal stem cells of step ii) may be derived from any one or more selected from the group consisting of umbilical cord, umbilical cord blood, placenta, amniotic membrane, bone marrow, fat, hair follicle, tooth, pulp and dermis. More preferably, the mesenchymal stem cells of step ii) may be umbilical cord-derived mesenchymal stem cells.

In the case of the umbilical cord-derived mesenchymal stem cells, by using the umbilical cord tissue discarded after childbirth, there is an advantage in that the collection is easy and a large amount of stem cells can be easily secured. Adipose or bone marrow-derived stem cells are subject to the age or health status of the donor from which they are separated and extracted, and thus have limitations and variability in proliferation, differentiation capacity and the like, but in the case of umbilical cord-derived stem cells, stem cells that can be obtained at the earliest time among adult stem cells are not almost affected by the ability of stem cells depending on variables such as the age of a donor, and have excellent proliferation and differentiation ability. In addition, the umbilical cord-derived mesenchymal stem cells have an advantage in that stem cell groups that can be used for various diseases such as neurological diseases, liver diseases, musculoskeletal diseases and the like can be isolated.

In step ii) above, the inoculation of the mesenchymal stem cells into the bubbles may be carried out when the air-permeable polymer membrane is coated with surfactant bubbles and then at least 2 hours or more elapse to initiate the disappearance of the bubbles.

The mesenchymal stem cells of step ii) may be stem cells subjected to 5 to 8 subcultures. More preferably, the mesenchymal stem cells may be stem cells subjected to 5 to 6 subcultures.

The mesenchymal stem cells inoculated in step ii) may be cultured in a culture medium, which may include any one or more selected from the group consisting of α-MEM, DMEM and FBS before being differentiated into osteoblasts in the differentiation medium, for 12 hours to 48 hours. More preferably, the mesenchymal stem cells may be cultured in the culture medium for 20 hours to 30 hours.

The differentiation of step iii) may be carried out for 24 hours to 120 hours. More preferably, the mesenchymal stem cells may be differentiated for 60 hours to 80 hours.

According to a preferred exemplary embodiment of the present invention, the osteoblasts may have higher expression levels of connexin 43 (CX43), Runt-related transcription factor 2 (RUNX2) and collagen type 1A1 (COL1A1) than undifferentiated stem cells and mature osteocytes; higher expression levels of angiopoietin 1 (ANGPT1) and alkaline phosphatase (AP) than undifferentiated stem cells; and lower expression levels of osterix (OSX), osteocalcin (OCN) and osteopontin (OPN) than mature osteocytes.

According to a preferred exemplary embodiment of the present invention, the osteoblasts may have a lower expression level of Ki-67 than undifferentiated stem cells.

It can be seen that the osteoblasts of the present invention are early osteoblasts that have significantly higher expressions of connexin 43 (CX43), Runt-related transcription factor 2 (RUNX2), collagen type 1A1 (COL1A1) and alkaline phosphatase (AP), which are expressed in early osteoblasts, than those of undifferentiated stem cells, and although the expression of Ki-67, which is a marker of cell proliferation, is decreased compared to that of undifferentiated cells, it is expressed, and cell proliferation occurs due to colony formation. In contrast, the expression levels of osterix (OSX), osteocalcin (OCN) and osteopontin, whose expressions increase as the osteoblasts sufficiently become mature osteocytes, are lower than those of mature osteocytes (NHOst). Moreover, the expression of angiopoietin 1 (ANGPT1), which is clearly expressed in early osteoblasts, is also increased several hundred times or more as the expression of genes and proteins, and thus, the characteristics of early osteoblasts are clearly shown (Table 1).

**[Table 1]**

| | Ki-67 | AP | ANGPT1 | RUNX2 | CX43 | COL1A | OSX | OPN | OCN |
|---|---|---|---|---|---|---|---|---|---|
| MSC | +++ | + | + | + | + | + | - | + | + |
| OB | + | +++ | +++ | ++++ | ++++ | ++++ | +++ | ++ | ++ |
| LONZA | (-) | (+) | (+) | ++ | ++ | ++ | +++ | +++ | +++ |

The CX43 is encoded by the GJA1 gene, and is the most common interstitial junction protein expressed in bone cell types such as chondrocytes, osteoblasts, osteocytes, osteoclasts or the like. CX43 plays an important role in regulating signal transduction between various bone cell types, and regulates bone development, differentiation, modeling and remodeling as well as pathology. In particular, CX43 is required for the survival, proliferation and differentiation of osteoblasts, and may improve the expressions of various bone morphogenetic markers.

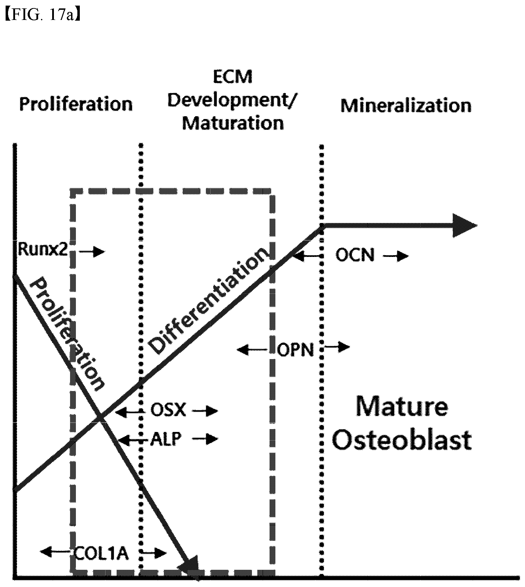

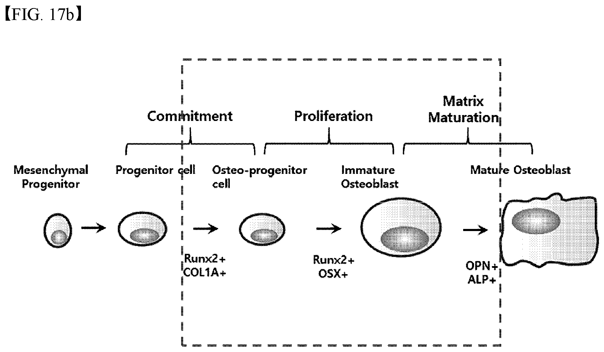

The RUNX2 is a major regulator of bone cell differentiation, and is an important transcription factor that regulates the expressions of alkaline phosphatase (ALP), which is an early phenotypic of osteoblast differentiation, and osteocalcin (OCN), which is a late phenotypic of osteoblast differentiation. In the early stage, immature osteoblasts (osteo-progenitors) are RUNX2+ and have proliferative capacity, differentiate into mature osteocytes and further undergo mineralization. Osteoblasts remain in a state where cell division does not stop for a certain period of time, and as differentiation progresses, they gradually lose their division ability and differentiate into osteocytes (FIGS. 17a and 17b).

The collagen type 1A1 (COL1A1) is a bone morphogenetic marker which is characteristically expressed when differentiated into osteoblasts.

The osteocalcin (OSX) corresponds to a differentiation factor associated with bone cell formation. OSX increases the expression of a bone mattress by increasing COL1A1 promoter activity, and plays an important role in differentiating immature osteoblasts into mature osteoblasts.

The osteocalcin (OCN) corresponds to a differentiation factor associated with bone cell formation. OCN is deposited in the bone matrix after being formed in osteoblasts, and then a portion of the newly formed is released into blood, and thus. the degree of bone formation may be known by measuring blood concentration.

The osteocalcin (OPN) is a differentiation factor associated with bone cell formation, and corresponds to a bone morphogenetic marker protein.

In addition, the present invention may provide a cell therapeutic agent for treating bone disease, including osteoblasts obtained by the method.

According to a preferred exemplary embodiment of the present invention, the osteoblasts may have higher expression levels of connexin 43 (CX43), Runt-related transcription factor 2 (RUNX2) and collagen type 1A1 (COL1A1) than undifferentiated stem cells and mature osteocytes; higher expression levels of angiopoietin 1 (ANGPT1) and alkaline phosphatase (AP) than undifferentiated stem cells; and lower expression levels of osterix (OSX), osteocalcin (OCN) and osteopontin (OPN) than mature osteocytes.

According to a preferred exemplary embodiment of the present invention, the osteoblasts may have a lower expression level of Ki-67 than undifferentiated stem cells.

According to a preferred exemplary embodiment of the present invention, the bone disease may be any one or more selected from the group consisting of fracture, femoral head bone necrosis, spinal union, delayed union or nonunion, osteoporosis, osteonecrosis, pseudoarthrosis, Paget's disease and osteodystrophy.

In addition, the present invention may provide a method for treating bone disease, including the step of administering osteoblasts obtained by the method to a patient with bone disease.

The administration may be parenteral administration, and the osteoblasts may be administered alone or in combination with methods using surgery, radiation therapy, hormone therapy, chemotherapy and a biological response modifier.

### [Advantageous Effects]

The differentiation method according to the present invention can stably and rapidly differentiate mesenchymal stem cells into osteoblasts. The differentiated osteoblasts have excellent blood vessel-forming ability and excellent bone-forming ability. Accordingly, the method for differentiating stem cells into osteoblasts and the osteoblasts obtained by the method, according to the present invention, can be effectively used as a cell therapeutic agent or treatment method related to bone disease.

### [Description of Drawings]

FIG. 1 shows a process for differentiating and obtaining umbilical cord-derived osteoblasts of the present invention.

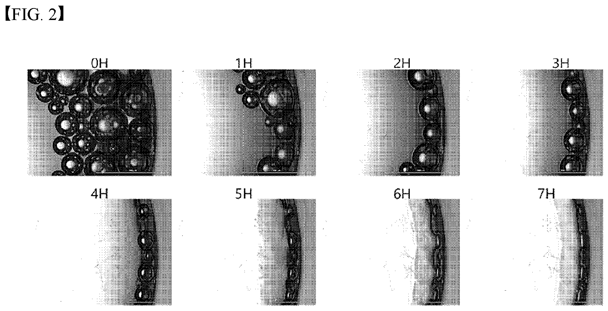

FIG. 2 shows the results of observing for 7 hours (0H to 7H) whether the poloxamer solution was removed after the poloxamer bubble coating and the poloxamer bubbles remaining on the porous membrane were maintained. It was confirmed that the bubbles disappeared after 2 hours (2H) after the removal of the poloxamer solution.

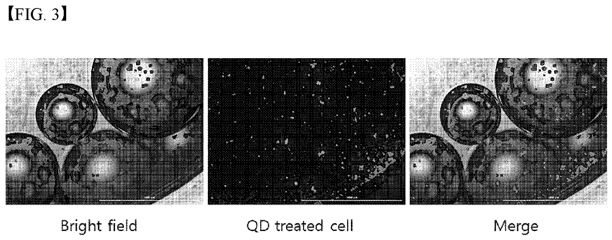

FIG. 3 shows the results of loading QD-treated cells after poloxamer bubble coating, which was obtained by photographing as a 3D image under a fluorescence microscope.

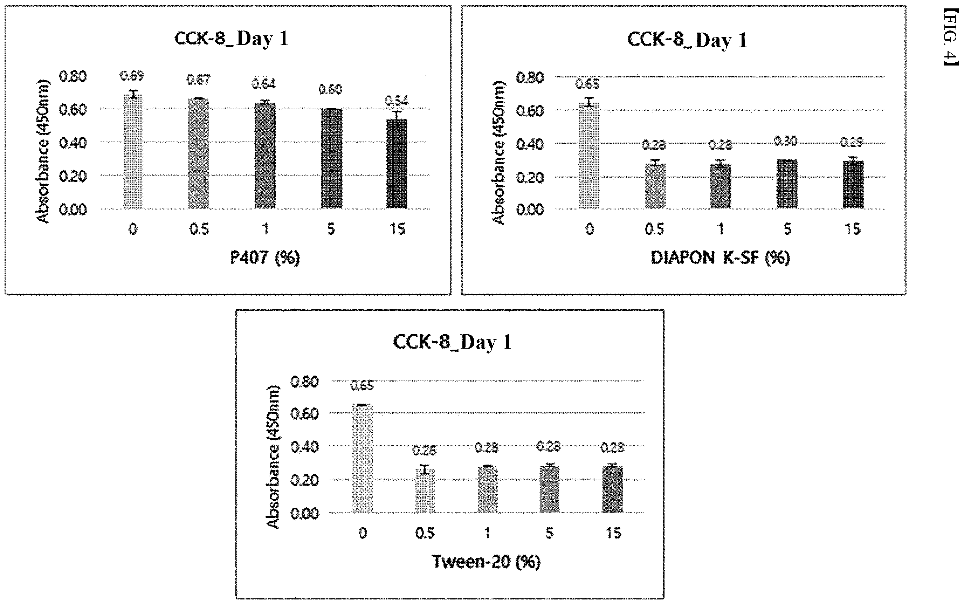

FIG. 4 shows the cytotoxicity test results for each type of surfactant. As a result of day 1 of CCK-8 (cell counting kit-8), it was confirmed that the remaining surfactants (DIAPON K-SF and Tween-20) except for P407 (poloxamer 407) were toxic to cells.

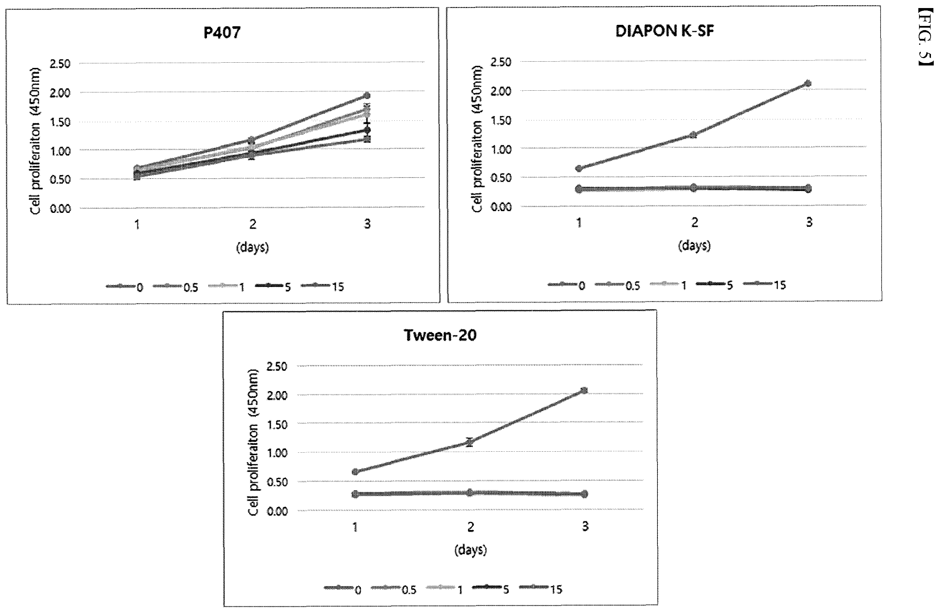

FIG. 5 shows the cytotoxicity test results for each type of surfactant. As a result of confirming cell proliferation from day 1 to day 3 of CCK-8 (cell counting kit-8), it was confirmed that the remaining surfactants (DIAPON K-SF and Tween-20) except for P407 (poloxamer 407) were cytotoxic, and cells did not proliferate.

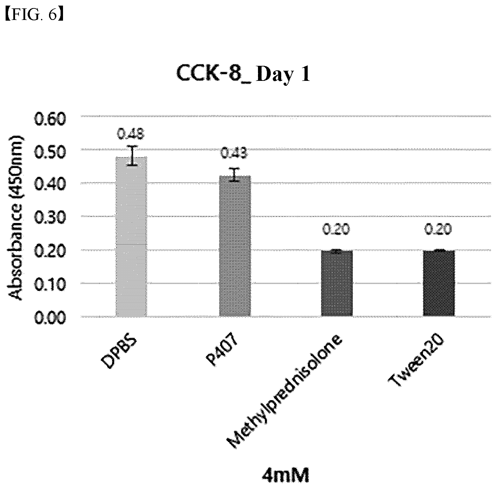

FIG. 6 shows the cytotoxicity test results for each type of surfactant. As a result of day 1 of CCK-8 (cell counting kit-8), it was confirmed that the remaining surfactants (methylprednisolone and Tween-20) except for P407 (poloxamer 407) were toxic to cells.

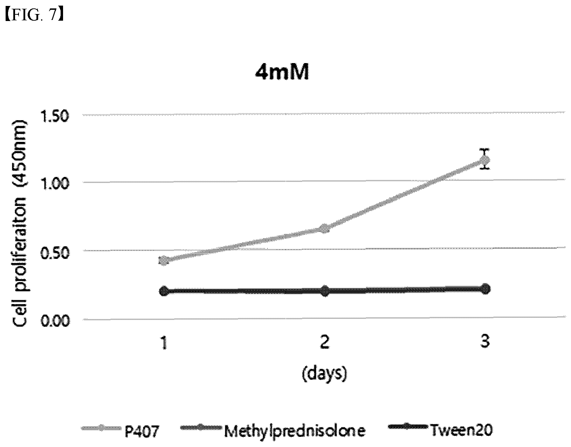

FIG. 7 shows the cytotoxicity test results for each type of surfactant. As a result of confirming cell proliferation from day 1 to day 3 of CCK-8 (cell counting kit-8), it was confirmed that the remaining surfactants (methylprednisolone and Tween-20) except for P407 (poloxamer 407) were cytotoxic, and cells did not proliferate.

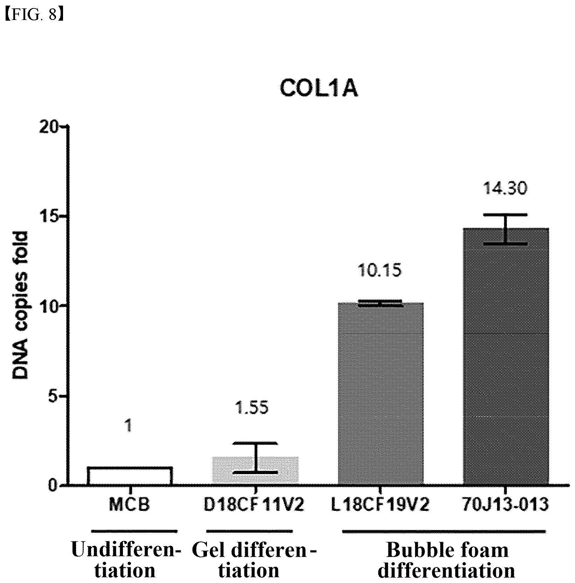

FIG. 8 shows that the gene (COL1A) expression level of cells differentiated in a bubble foam surfactant was significantly higher than in undifferentiated cells or cells differentiated in gel surfactant.

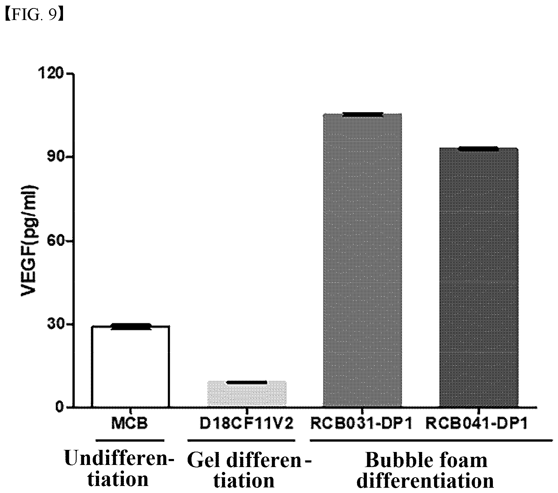

FIG. 9 shows that the protein (VEGF) expression level of cells differentiated in a bubble foam surfactant was significantly higher than in undifferentiated cells or cells differentiated in a gel surfactant.

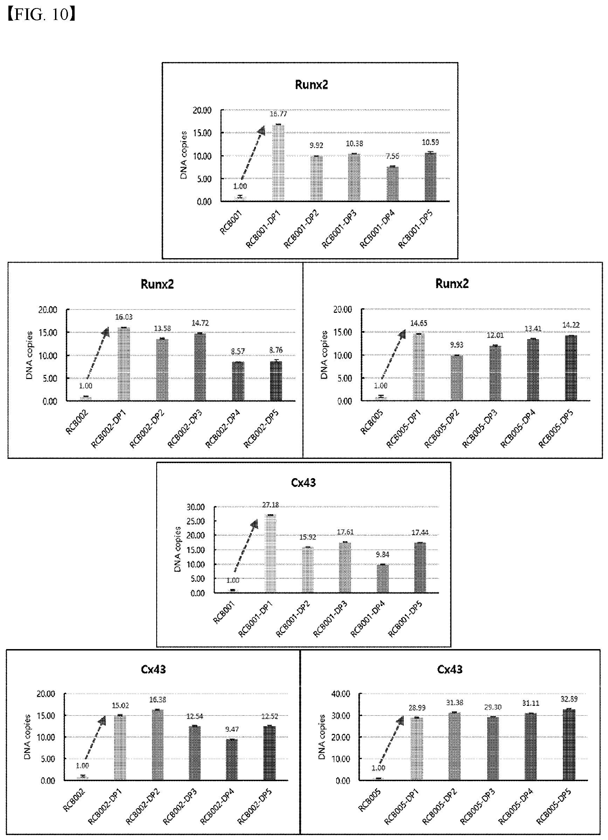

FIG. 10 shows the results of comparing the expression levels of the bone induction gene of the differentiated cell therapeutic agents (RCB001-DP1 to RCB005-DP5) of the present invention compared to the undifferentiated cells (RCB001, RCB002, RCB005). It was confirmed that the expression levels of connexin 43 (CX43) and Runt-related transcription factor 2 (RUNX2) secreted from the cell therapeutic agents of the present invention were significantly increased compared to undifferentiated cells.

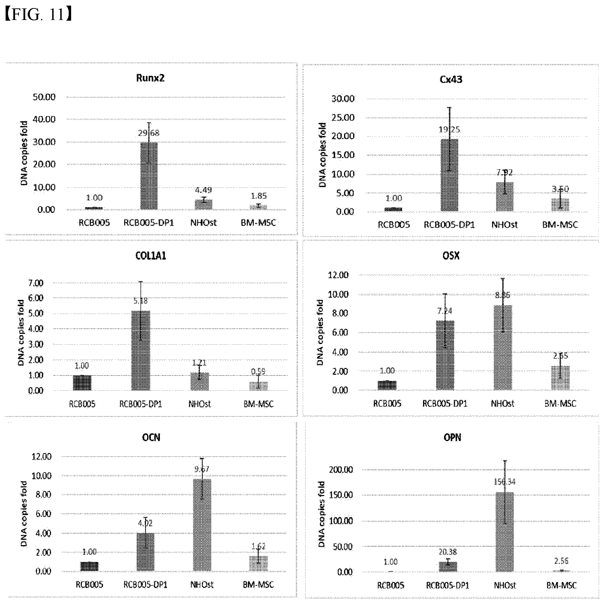

FIG. 11 shows the results of comparing the expression levels of the bone inducing genes of the differentiated cell therapeutic agent (RCB005-DP1) of the present invention with bone marrow-derived mesenchymal stem cells (BM-MSC) and mature osteoblasts (NHOst) compared to the undifferentiated cells (RCB005). It was confirmed that the osteoblasts had higher expression levels of connexin 43 (CX43), Runt-related transcription factor 2 (RUNX2) and collagen type 1A1 (COL1A1) which are the early osteoblast markers, compared to the undifferentiated and mature osteoblasts, and the expression levels of osterix (OSX), osteocalcin (OCN) and osteopontin (OPN) were lower than those of the mature osteoblasts (NHOst).

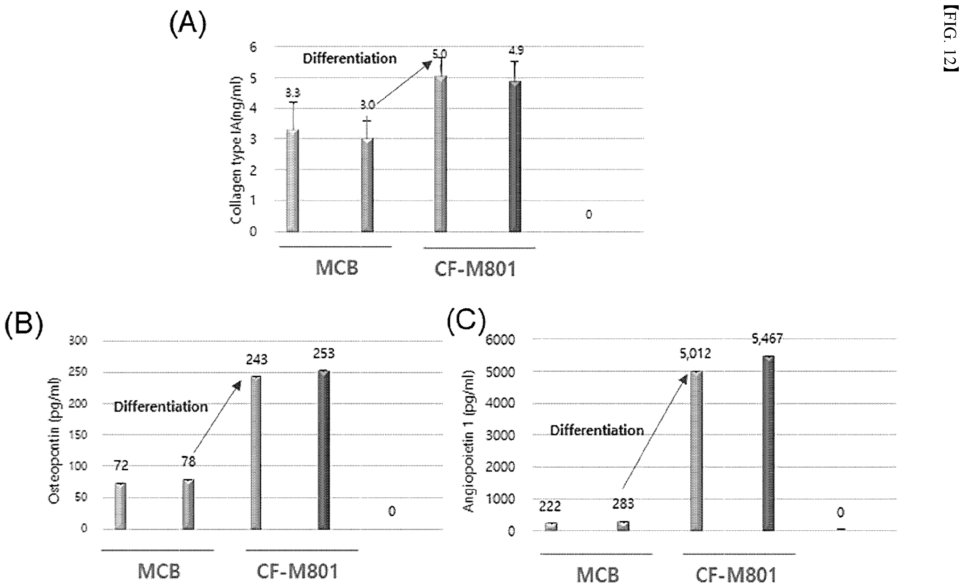

FIG. 12 confirms that the osteogenic marker protein and the vascular inducing marker protein were increased in the differentiated osteocyte therapeutic agent of the present invention compared to the undifferentiated cells. (A) represents COL1A1, (B) represents Osteopontin (OPN), and (C) represents Angiopoietin.

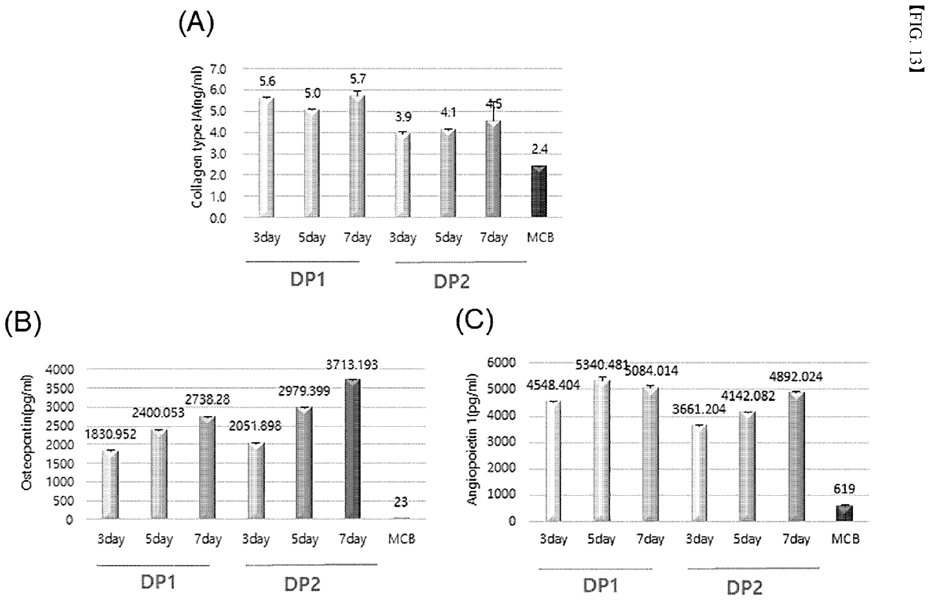

FIG. 13 shows that the differentiated cell therapeutic agent of the present invention maintained the osteogenic marker protein and the vascular inducing marker protein even after day 3 to day 7 after thawing. (A) represents COL1A1, (B) represents osteopontin (OPN), and (C) represents angiopoietin.

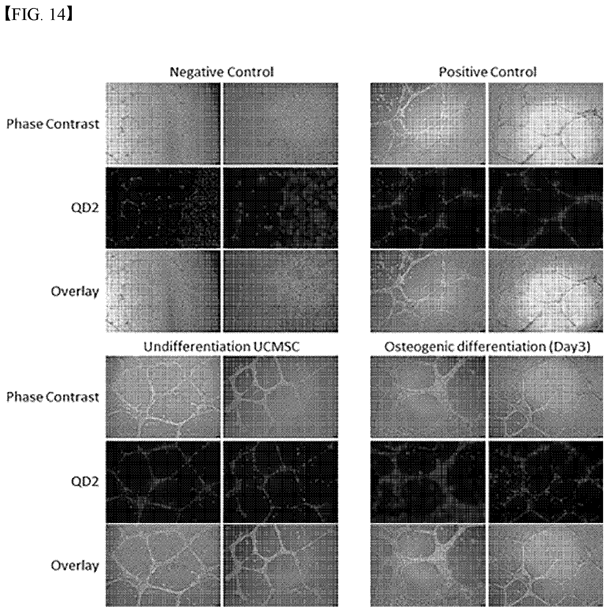

FIG. 14 shows the vascular endothelial angiogenic ability of the umbilical cord-derived osteoblasts of the present invention. It was confirmed that, the umbilical cord-derived source cells (Undifferentiation UCMSC) and the osteogenic differentiation cell therapeutic agent were induced to produce blood vessels as much as the VEGF positive control known as the vascular angiogenic inducing factor, and in particular, the blood vessels formed by the proteins secreted by the cell therapeutic agent were formed to be thicker and stronger.

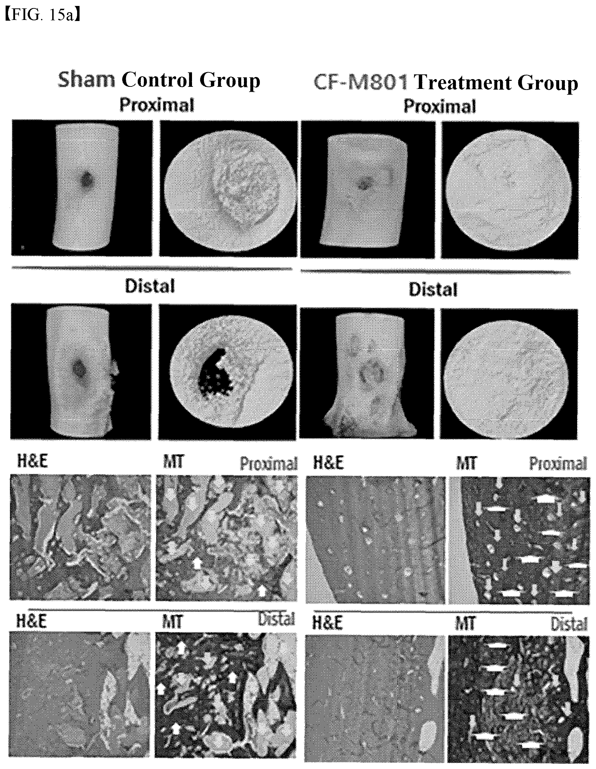

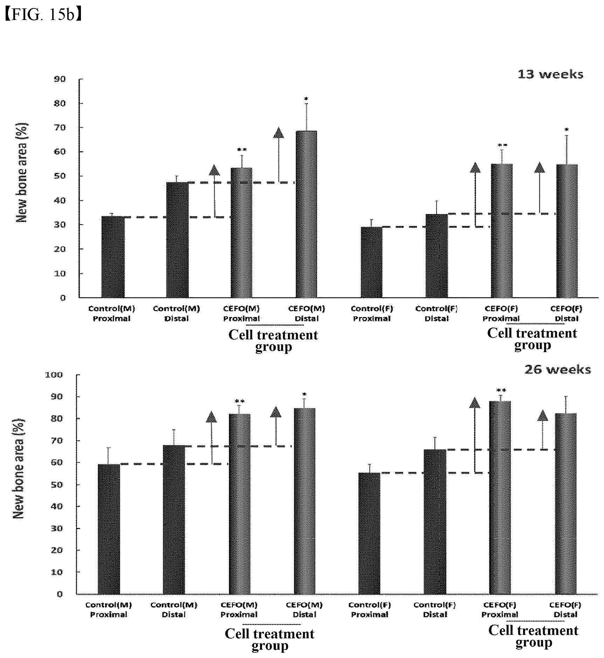

FIGS. 15a and 15b show the results of the effectiveness of the cell therapeutic agent of the present invention against a large animal (goat).

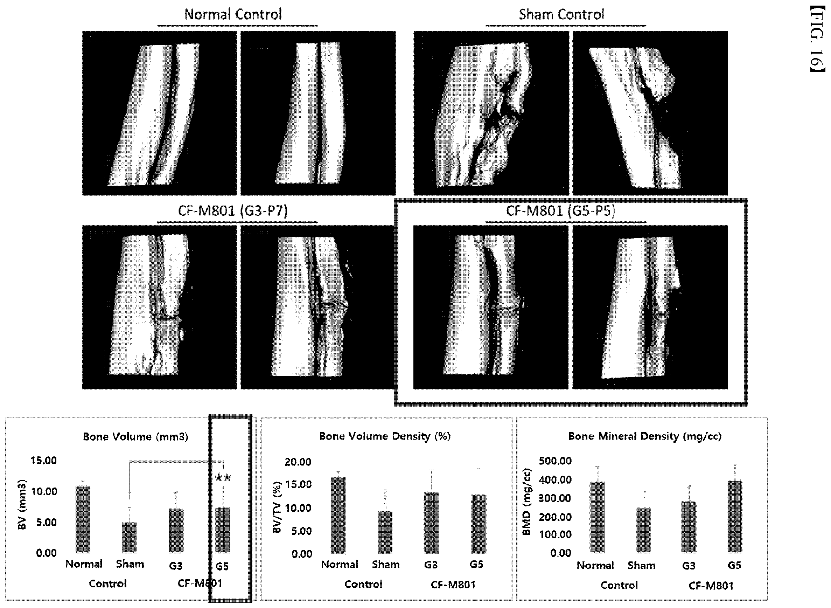

FIG. 16 shows the results of the effectiveness of the cell therapeutic agent of the present invention against a small animal (rat).
FIGS. 17a and 17b show the differentiation step from mesenchymal stem cells into osteocytes. The region indicated by the dotted line indicates the step of the cell therapeutic agent of the present invention.

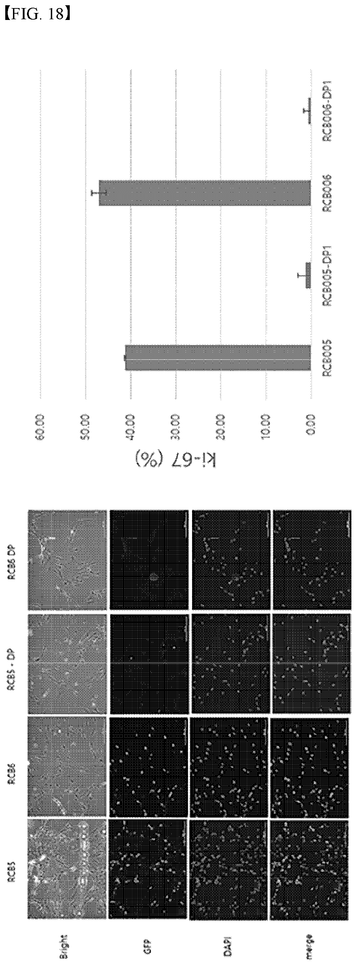

FIG. 18 shows the results of confirming the cell mitogenic ability of the undifferentiated UCMSC (source) and the differentiated osteoblasts (DP) by Ki-67 (marker of cell division). Consistently, it was confirmed that the expression of Ki-67 rapidly decreased to 1% or less in DP, which means that CF-M801 is the osteoblasts in the early stage of the osteogenic model.

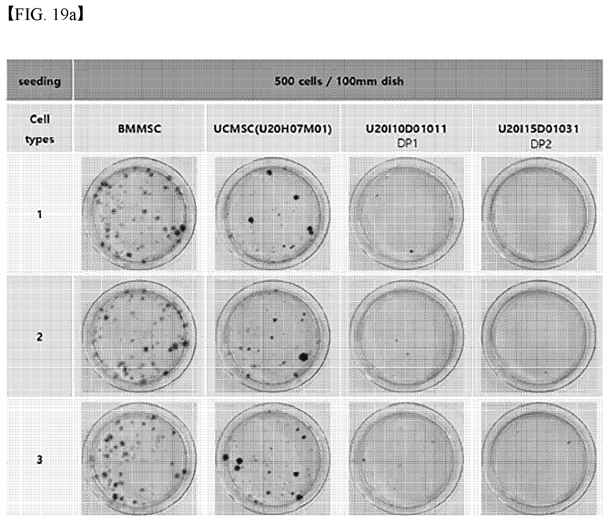

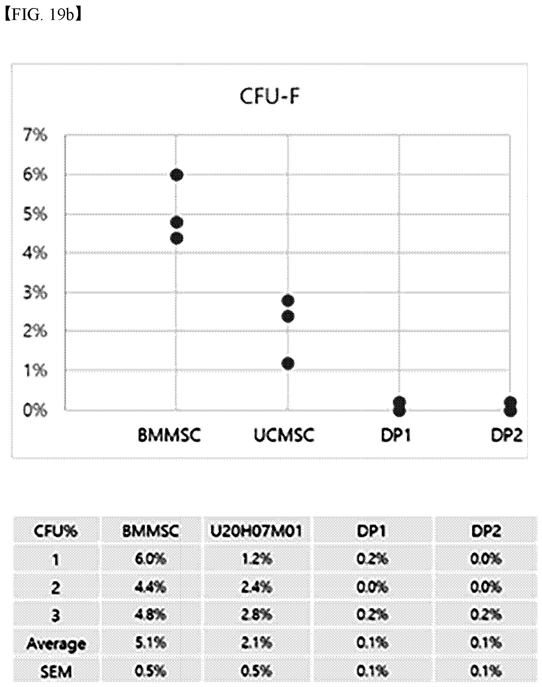

FIGS. 19a and 19b show the results of measuring CFU-F by using 2 batches of the undifferentiated stem cells BMMSC and UCMSC (source) and the differentiated osteoblasts (DP). It was confirmed that the CFU-F of the differentiated osteoblasts (DP, CF-M801) decreased in a pattern similar to the expression of Ki-67 (marker of cell division).

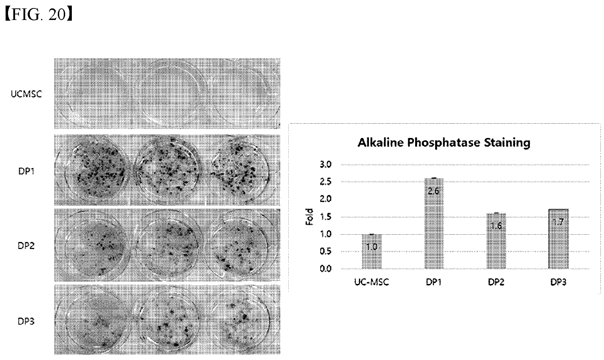

FIG. 20 shows the results of measuring the absorbance by staining with alkaline phosphatase after forming colonies by using 2 batches of the undifferentiated stem cells UCMSC (source) and the differentiated osteoblasts (DP). The colonies made by differentiated osteoblasts (DP) were stained with alkaline phosphatase and the absorbance value increased compared to undifferentiated cells, and thus, it was confirmed that the colonies produced by the proliferation of the present cell therapeutic agents were osteoblasts.

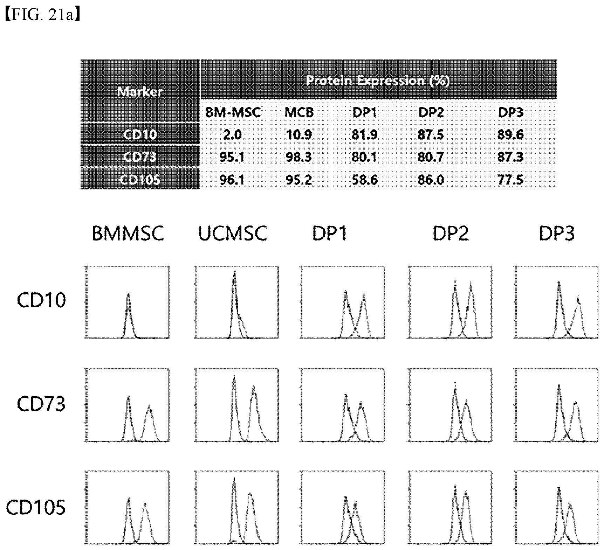

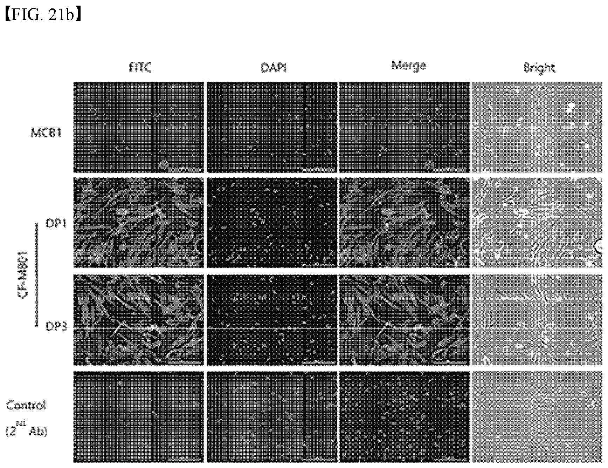

FIGS. 21a and 21b show that the differentiated osteoblasts (DP1 to DP3) of the present invention expressed 80% or more of CD10, whereas the undifferentiated mesenchymal stem cells expressed 15% or less of CD10. This means that the osteoblasts of the present invention were managed by checking the purity by confirming the osteoblast-specific markers, not the existing stem cell markers.

### [Best Mode]

### [Example 1]

### Isolation and harvesting of umbilical cord-derived stem cells

First, arterial and venous blood vessels were removed from a separated umbilical cord, and the remaining tissue was minced and reacted with AdiCol^{™} (CEFO) at 37°C for 30 minutes or more, and then cells were extracted. The extracted cells were cultured in CEFOgro^{™} medium under conditions of 37°C and 5% CO₂ to obtain mesenchymal stem cells.

### [Example 2]

### Coating air-permeable polymer membrane with surfactant bubbles

On the porous membrane of a hyper flask, 8% poloxamer 407 dissolved in PBS was shaken to generate bubbles, and the remaining poloxamer solution was poured out. The air-permeable polymer membrane was coated with poloxamer bubbles at 37°C for 2 hours. After removing the poloxamer 407 bubbles through microscopic 3D imaging, the solution was removed, and as a result of observing whether the bubbles were maintained, the bubbles started to disappear after 2 hours (2H), and after 5 hours (5H), it was confirmed that the bubbles completely disappeared (FIG. 2).

In addition, quantum dot-conjugated silica nanoparticles (QD) were uptaken into stem cells for 24 hours to track umbilical cord-derived mesenchymal stem cells. After the poloxamer 407 bubbles were generated, the solution was removed, and the cells were loaded and then imaged in 3D by using a microscope Z-stack (FIG. 3).

### [Example 3]

### Differentiation into osteoblasts and culture

After 2 hours after coating with poloxamer bubbles in [Example 2], the \umbilical cord-derived stem cells obtained in [Example 1] were inoculated at a density of 1 × 10⁴ cells/cm², and the cells were cultured in a culture medium including DMEM and FBS for 24 hours. The cultured stem cells were differentiated into osteoblasts for 72 hours in the bone differentiation medium.

### [Example 4]

### Selection of suitable surfactants for coating air-permeable polymer membranes

The toxicity of poloxamer (poloxamer 407, p407), sodium methyl cocoyl taurate (DIAPON K-SF), polyoxyethylene sorbitan monolaurate (Tween 20) or methylprednisolone, which are known as representative biocompatible surfactants, was confirmed.

Specifically, in the upper chamber in a transwell including a three-dimensional porous membrane, the surfactant p407, DIAPON K-SF or Tween 20 was respectively treated at 0, 0.5 1, 5 or 15% (v/v) and coated for 2 hours in the same manner as in [Example 2], and then, the umbilical cord-derived mesenchymal stem cells obtained in [Example 1] were seeded at 20,000 cells/cm². The cell growth medium was placed in the lower chamber and cultured for 3 days. On days 1, 2 and 3, the CCK-8 (Cat. CK04, DOJINDO) solution was added, and after 3 hours of reaction in a 37°C CO₂ incubator, absorbance was measured at 450 nm to analyze cytotoxicity (FIG. 4) and the degree of cell growth (FIG. 5).

Further, in a transwell including a three-dimensional porous membrane, the surfactant p407, methylprednisolone or Tween 20 was treated at a concentration of 4 mM in the upper chamber, and after coating for 2 hours in the same manner as in [Example 2], the umbilical cord-derived mesenchymal stem cells obtained in [Example 1] were seeded at 20,000 cell/cm². The cell growth medium was placed in the lower chamber and cultured for 3 days. On days 1, 2 and 3, the CCK-8 solution was added, and after 3 hours of reaction in a 37°C CO₂ incubator, absorbance was measured at 450 nm to analyze cytotoxicity (FIG. 6) and the degree of cell growth (FIG. 7).

As a result, it was confirmed that all of the remaining DIAPON K-SF, Tween 20 and methylprednisolone except for P407 were toxic to cells, as shown in [FIG. 4] to [FIG. 7].

### [Example 5]

### Selection of surfactant coating method

After coating poloxamer in a bubble or gel state on a hyper flask porous membrane, the degree of osteodifferentiation of umbilical cord-derived stem cells differentiated by the method of [Example 3] was compared.

Specifically, differentiation-induced osteoblasts and undifferentiated cells were respectively collected, treated with Trizol^{™} and chloroform (Sigma) and separated by centrifugation to obtain only mRNA. The obtained mRNA was synthesized as cDNA by using the Transcriptor Universal cDNA Master Kit (Roche). Thereafter, DNA was amplified through RT-PCR for COL1A1 to confirm the difference in the numbers of DNA copies at the gene level. Polymerase chain reaction conditions were 50 cycles of 95°C for 10 seconds, 54°C for 10 seconds and 72°C for 30 seconds (FIG. 8).

In addition, by collecting the culture medium of osteoblasts and undifferentiated cells prepared and differentiated by the method of [Example 3], the degree of secretion of vascular endothelial growth factor (VEGF) was determined by performing enzyme-linked immunosorbent assay (ELISA) (FIG. 9).

As a result, as shown in [FIG. 8] and [FIG. 9], it was confirmed that the expression levels of osteoinduction genes and angiogenesis-inducing proteins in cells differentiated by the method of the present invention were more excellent than in cells differentiated from undifferentiated or gel-type poloxamer.

### [Example 6]

### Evaluation of osteogenic ability of obtained osteoblasts

### <6-1> Osteogenic ability of osteoblasts (gene level)

After synthesizing cDNA by separating RNA from osteoblasts collected during the differentiation process of osteoblasts, the gene expression levels of Runt-related transcription factor 2 (RUNX2) and connexin 43 (CX43), which are osteogenic gene markers, were compared with those of undifferentiated cells by using real time-polymerase chain reaction (RT-PCR).

Specifically, osteoblasts and undifferentiated cells prepared and differentiated by the method of [Example 3] were respectively collected, treated with Trizol^{™} and chloroform (Sigma) and separated by centrifugation to obtain only mRNA. The obtained mRNA was synthesized as cDNA by using the Transcriptor Universal cDNA Master Kit (Roche). Thereafter, DNA was amplified through RT-PCR targeting RUNX2 and CX43 to determine the difference in expression levels at the gene level. Polymerase chain reaction conditions were 50 cycles of 95°C for 10 seconds, 54°C for 10 seconds and 72°C for 30 seconds.

In addition, the mRNA was obtained by respectively collecting osteoblasts, undifferentiated cells, bone marrow-derived mesenchymal stem cells (BM-MSC) and mature osteocytes (NHOst) prepared and differentiated by the method of [Example 3], and the obtained mRNA was synthesized as cDNA by using the Transcriptor Universal cDNA Master Kit (Roche). Thereafter, DNA was amplified through RT-PCR for RUNX2, CX43 and COL1A to confirm the difference in expression levels at the gene level. Polymerase chain reaction conditions were 50 cycles of 95°C for 10 seconds, 54°C for 10 seconds and 72°C for 30 seconds.

As a result, as shown in [FIG. 10] and [FIG. 11], it was confirmed that RUNX2, CX43 or COL1A was highly expressed in osteoblasts of the present invention compared to undifferentiated stem cells and mature osteocytes (NHOst). In [FIG. 10], RCB001, RCB002 and RCB005 refer to undifferentiated cells, and RCB001-DP1 to RCB005-DP5 refer to the differentiated cell therapeutic agents of the present invention.

On the other hand, OSX, OCN or OPN was expressed higher in mature osteoblasts (NHOst) than in the osteoblasts of the present invention, indicating that the osteoblasts of the present invention were in the immature osteodifferentiation stage compared to NHOst (FIG. 11). In [FIG. 11], RCB001, RCB002 and RCB005 refer to undifferentiated cells, and RCB001-DP1 to RCB005-DP5 refer to the differentiated cell therapeutic agents of the present invention.

### <6-2> Osteogenesis ability of osteoblasts (protein level)

The change in the protein concentrations of collagen type 1A1 (COL1A), osteopontin or angiopoirtin (ANGPT-1), which are known as osteogenic or angiogenic protein markers, was confirmed in the culture medium and osteoblasts collected in the osteoblast differentiation process of [Example 3]. Additionally, it was attempted to determine whether the frozen cell therapeutic agent of the present invention could maintain bone morphogenetic proteins or vascular-inducing proteins even after thawing.

Specifically, the prepared and differentiation-induced osteoblasts, undifferentiated cells and the differentiation medium prepared and differentiated by the method of [Example 3] were respectively collected to perform enzyme-linked immunosorbent assay (ELISA). For COL1A, the degree of change in protein expression in the cells in which the cells were lysed was confirmed by taking differentiation-induced cells and undifferentiated cells, and for osteopontin and angiopoietin, the protein secretion levels were determined in the culture medium up to 72 hours of differentiation.

As a result, as shown in [FIG. 12], it was confirmed that the protein expression of COL1A was increased and the secretions of osteopontin and angiopoietin were all highly expressed in the osteoblasts of the present invention. In addition, as shown in [FIG. 13], it was confirmed that the bone-forming proteins or the vascular-inducing proteins of the cell therapeutic agent of the present invention was maintained even after 3 to 7 days after thawing. In [FIG. 12] and [FIG. 13], (A) denotes COL1A1, (B) denotes osteopontin (OPN) and (C) denotes angiopoietin.

### [Example 7]

### Evaluation of angiogenic ability of obtained osteoblasts

The angiogenic ability of human umbilical vein endothelial cells (HUVEC) due to the angiogenic protein secreted by the obtained osteoblasts was confirmed.

Specifically, undifferentiated stem cells or osteoblasts were inoculated at 4 × 10⁴ cells/well on a 12-well transwell plate including a porous membrane with 8.0 µm pore size, and quantum-dot was uptaken for 24 hours by using HUVEC cells in culture, and then, the cells were taken and inoculated at a density of 25,000/cm² under the transwell. After inoculation, co-culture was carried out for 1 day to conduct the vascular tube formation analysis experiment for HUVEC. After seeding each cell in a 12-well plate, medium and substance exchange was allowed to occur by using a transwell, and then, the cells were cultured for 12 hours to confirm the vascular induction of HUVEC cells. Osteogenic differentiated cells and undifferentiated cells were placed on the transwell for comparison. The negative control group was inoculated with only HUVEC cells on the coated Matrigel and cultured for 12 hours by using the HUVEC medium without VEGF, and the positive control group was cultured with 20 ng/mL of VEGF added under the same conditions as the negative control group.

As a result, as shown in [FIG. 14], when co-cultured with umbilical cord-derived source cells (Undifferentiation UC-MSC) and the bone cell therapeutic agent to a similar level with the VEGF-positive control, which is known as an angiogenesis-inducing factor, it was confirmed that tube formation occurred. In addition, it was confirmed that thick and strong blood vessels were formed when co-cultured with the osteogenic differentiation cell therapeutic agent, and in particular, blood vessels formed by the protein secreted by the cell therapeutic agent were thicker and stronger.

### [Example 8]

### Confirmation of bone regeneration ability of obtained osteoblasts - in vivo

After inducing bone defects in a large animal (goat) or small animal (rat) model, it was attempted to determine the bone regeneration ability of the cell therapeutic agent of the present invention.

Specifically, after inducing a femoral bone defect in an immunosuppressed goat model, 1 × 10⁷ osteoblasts were treated per goat to confirm the bone regeneration effect for 26 weeks (Table 2). In addition, as an efficacy test for the goat model, bone tissue was demineralized for two and a half months, and histological evaluation of the efficacy of this cell therapeutic agent was carried out with H&E and Masson's Trichrome staining.

In the case of the immunosuppressed rat model, after inducing a radial bone defect, 1 × 10⁶ osteoblasts per rat were treated to confirm the effect of bone regeneration for 12 weeks (Table 3). The Sham control group was treated only with scaffolds composed of alginate. The degree of bone regeneration was confirmed by µCT image, and bone tissue was fixed, demineralized and sectioned to prepare slides, and after staining, new bone regeneration was confirmed.

**[Table 2]**

| **Group** | **Administered substance** | **Number of animals (M/F)** | **Route** | **Dose** |
|---|---|---|---|---|
| Sham control group | - | 6/6 | Femoral bone defect | - |
| Test substance administration group | CF-M801 | 8/8 | Femoral bone defect | 1 × 10⁷ |

**[Table 3]**

| **Group** | **Administered substance** | **Number of animals (M/F)** | **Route** | **Dose** |
|---|---|---|---|---|
| Sham control group | - | 10 | Radial bone defect | - |
| Test substance administration group | CF-M801 | 10 | Radial bone defect | 1 × 10⁶ |

As a result of the efficacy test for the goat model, as shown in [FIG. 15a] and [FIG. 15b], it was confirmed that in both males and females, the formation of new bone in the proximal and distal regions of the control group was very poor and only partially confirmed at 26 weeks, and it was confirmed that they were in the process of wound healing. On the other hand, in the proximal and distal parts of the cell-administered group, cells filled the defect site, proliferated and differentiated into osteocytes such that the new bone was well connected organically without breaking, and it was confirmed that the thickness was considerably increased (lower left white arrow in FIG. 15a). Blood vessels were also found to be well formed regularly. It was confirmed that the formation of new bone was significantly observed at both 13 and 26 weeks only in the cell-administered group.

As a result of the efficacy test on the rat model, as shown in [FIG. 16], the G3 group was a cell therapeutic agent group differentiated at passage 7 for 2 days, and the G5 group was a treatment group differentiated for 3 days at passage 5, and the volume of the bone, the volume density of bone and BMD tended to increase in both groups compared to the control group, and particularly, it was confirmed that the bone volume of the therapeutic agent group differentiated from passage 5 for 3 days increased more significantly.

### [Example 9]

### Confirmation of stage of cell therapeutic agent

It was intended to determine the stage of the cell therapeutic agent of the present invention.

### <9-1> Confirmation of Ki-67 expression

It is known that human Ki-67 is not expressed in the stationary phase (G0) of the cell cycle but is expressed in the proliferation phase (G1, S, G2, M phase), and the cell therapeutic agent is not expressed because cells do not differentiate after differentiating into osteocytes. Accordingly, in two batches of the undifferentiated UCMSC (source) and differentiated osteoblasts (DP) of the present invention, the expression level of Ki-67, which is an indicator of cell division, was confirmed by immunocytochemistry (ICC), and DAPI, which is a nuclear staining material, was stained to correct the expression level of Ki-67 compared to the number of cells.

Specifically, a slide plate was prepared, and undifferentiated and differentiated cells were inoculated into each well in 3 × 10⁵ doses, and then cultured in a CO₂ incubator for 24 hours. Cells were fixed with 4% formaldehyde at room temperature for 10 minutes, and cells were permeabilized with 1% Triton X-100 for 10 minutes at room temperature. After blocking with BSA at room temperature for 30 minutes, the Ki-67 primary antibody was reacted at room temperature for 1 hour, and the fluorescence-linked secondary antibody was reacted at room temperature for 1 hour after blocking light. Mounting was performed by using ProLong^{™} Gold Antifade Mountant (Invitrogen) containing DAPI, and cells were observed by using a fluorescence microscope.

As a result, as shown in [FIG. 18], it was consistently confirmed that the expression of Ki-67 rapidly decreased to 1% or less in osteoblasts (DP).

### <9-2> Confirmation of CFU-F expression

CFU-F was measured by using two batches of undifferentiated stem cells BMMSC, UCMSC (source) and differentiated osteoblasts (DP) of the present invention. In addition, it is known that mesenchymal stem cells start to proliferate and form characteristic cell colonies when *in vitro* culture is started, and the cells in the colony look like fibroblasts, and each cell colony is called a colony forming unit-fibroblast, which is known as an important feature of stem cells. Accordingly, the formed colonies were observed with the naked eye and a camera photograph after 2% crystal violet staining was performed, and the dyed colonies were quantified by counting those with 3 mm or more and a density of 80% or more.

As a result, as shown in [FIG. 19a] and [FIG. 19b], it was confirmed that the CFU-F of the differentiated osteoblasts (DP, CF-M801) decreased in a pattern similar to the expression of Ki-67 (indicator of cell division) expression. In addition, it was confirmed that a large number of colonies were formed in bone marrow-derived stem cells and source cells, which are umbilical cord-derived stem cells, but almost no colony formation occurred in the cell therapeutic agent. From this, it was determined that the umbilical cord-derived stem cell source cells were differentiated into osteogenic differentiated cells, and thus lost the ability to form colonies. Through these results, it was confirmed that most of the cell therapeutic agents were differentiated from the source cells.

### <9-3> Confirmation of ALP expression

The purpose of this study was to determine whether the colony-forming cells, which appeared at an average of less than 1% in CFU-F using the cell therapeutic agent CF-M801, were undifferentiated mesenchymal stem cells or osteoblasts induced to differentiate.

Specifically, colonies that appeared after culturing in the same manner as CFU-F in Example <9-2> were stained with alkaline phosphatase (ALP) staining to determine whether or not bone differentiation occurred. After inoculating 10,000 cells and maintaining them for a week, ALP staining was performed. After ALP staining, dimethylsulfoxide was reacted to dissolve the stain, and only the supernatant was taken to measure the absorbance with a microplate reader.

As a result, as shown in [FIG. 20], undifferentiated UCMSCs, which are allogeneic umbilical cord-derived mesenchymal stem cells, showed little ALP staining, but osteodifferentiation-induced CF-M801 colonies were all ALP-positive in three different lots. In addition, the osteodifferentiation-induced CF-M801 showed higher absorbance compared to the undifferentiated cells. As a result of analyzing these by converting into relative values, it showed a value 1.5 times higher than that of undifferentiated UCMSC in all cases. As a result, it was confirmed that the differentiated osteoblasts (DP) were in a state where cell division did not stop for a certain period of time, and as the differentiation progressed, they gradually lost their dividing ability and differentiated into osteocytes.

In summary, the cell therapeutic agent of the present invention may differentiate mesenchymal stem cells into osteoblasts by RUNX2, which is a master regulator of osteocyte differentiation. It was found that early-stage osteoblasts (osteo-progenitors, immature osteoblasts) are RUNX2+, have proliferative capacity, differentiate into mature osteocytes, and further progress in mineralization (FIGS. 17a and 17b).

### <9-4> Confirmation of CD-10 expression

In order to confirm the differentiation of the cell therapeutic agents, the expression level of CD10 was determined by flow cytometry (FACS) and immunocytochemistry (ICC).

Specifically, osteoblasts, undifferentiated cells and bone marrow-derived mesenchymal stem cells (BM-MSC) prepared and differentiated by the method of [Example 3] were suspended in a 2% BSA/DPBS solution. The CD10 primary antibody was reacted at room temperature for 1 hour, and after washing, the FITC fluorescence-linked secondary antibody was reacted at room temperature for 30 minutes. After washing the cells, the supernatant was removed, 3.7% formaldehyde was added and fixed at room temperature for 20 minutes, and the expression level of CD10 was determined by using a flow cytometer (BD Accuri C6 Plus).

For ICC, a slide plate was prepared, and after inoculating undifferentiated and differentiated cells in 3 × 10⁵ doses in each well, the cells were cultured in a CO₂ incubator for 24 hours. The cells were fixed with 4% formaldehyde at room temperature for 10 minutes, and the cells were permeabilized with 1% Triton X-100 for 10 minutes at room temperature. After blocking with BSA at room temperature for 30 minutes, the CD10 primary antibody was reacted at room temperature for 1 hour, and the FITC fluorescence-linked secondary antibody was reacted at room temperature for 1 hour after blocking light. Mounting was performed by using ProLong^{™} Gold Antifade Mountant (Invitrogen) containing DAPI, and the cells were observed using a fluorescence microscope.

As a result, as shown in [FIG. 21a] and [FIG. 21b], it was confirmed that the differentiated osteoblasts (DP1 to DP3) of the present invention expressed 80% or more of CD10, whereas the undifferentiated mesenchymal stem cells expressed 15% or less of CD10. This means that the osteoblasts of the present invention were managed by checking the purity by confirming the osteoblast-specific markers, not the existing stem cell markers.

### [Industrial Applicability]

The differentiation method according to the present invention can stably and rapidly differentiate mesenchymal stem cells into osteoblasts. The differentiated osteoblasts have excellent blood vessel-forming ability and excellent bone-forming ability. Accordingly, the method for differentiating stem cells into osteoblasts and the osteoblasts obtained by the method, according to the present invention, can be effectively used as a cell therapeutic agent or treatment method related to bone disease.

## Claims

1. A method for differentiating mesenchymal stem cells into osteoblasts, comprising the steps of:
i) coating an air-permeable polymer membrane with surfactant bubbles;
ii) inoculating mesenchymal stem cells into the bubbles of step i) at a density of 1 × 10³ to 1 × 10⁵ cells/cm²;
iii) differentiating the mesenchymal stem cells of step ii) into osteoblasts in a differentiation medium; and
iv) isolating and obtaining osteoblasts differentiated in step iii).

2. The method of claim 1, wherein the surfactant of step i) is a poloxamer.

3. The method of claim 1, wherein the bubbles of step i) are produced by comprising the step of:
generating bubbles by adding a surfactant on the air-permeable polymer membrane and moving.

4. The method of claim 1, wherein the mesenchymal stem cells of step ii) are derived from any one or more selected from the group consisting of umbilical cord, umbilical cord blood, placenta, amniotic membrane, bone marrow, fat, hair follicle, tooth, pulp and skin dermis.

5. Osteoblasts obtained by the method of claim 1.

6. The osteoblasts of claim 5, wherein the osteoblasts have higher expression levels of connexin 43 (CX43), Runt-related transcription factor 2 (RUNX2) and collagen type 1A1 (COL1A1) than undifferentiated stem cells and mature osteocytes; higher expression levels of angiopoietin 1 (ANGPT1) and alkaline phosphatase (AP) than undifferentiated stem cells; and lower expression levels of osterix (OSX), osteocalcin (OCN) and osteopontin (OPN) than mature osteocytes.

7. The osteoblasts of claim 5, wherein the osteoblasts have a lower expression level of Ki-67 than undifferentiated stem cells.

8. A cell therapeutic agent for treating bone disease, comprising osteoblasts obtained by the method of claim 1.

9. The cell therapeutic agent of claim 8, wherein the osteoblasts have higher expression levels of connexin 43 (CX43), Runt-related transcription factor 2 (RUNX2) and collagen type 1A1 (COL1A1) than undifferentiated stem cells and mature osteocytes; higher expression levels of angiopoietin 1 (ANGPT1) and alkaline phosphatase (AP) than undifferentiated stem cells; and lower expression levels of osterix (OSX), osteocalcin (OCN) and osteopontin (OPN) than mature osteocytes.

10. The cell therapeutic agent of claim 8, wherein the osteoblasts have a lower expression level of Ki-67 than undifferentiated stem cells.

11. The cell therapeutic agent of claim 8, wherein the bone disease is any one or more selected from the group consisting of fracture, femoral head bone necrosis, spinal union, delayed union or nonunion, osteoporosis, osteonecrosis, pseudoarthrosis, Paget's disease and osteodystrophy.

12. A method for treating bone disease, comprising the step of:
administering osteoblasts obtained by the method of claim 1 to a patient with bone disease.
